Europäisches Patentamt

European Patent Office

Office européen des brevets

(19)

(11) Publication number: **0 141 514**
**A2**

(12)

# EUROPEAN PATENT APPLICATION

(21) Application number: **84306187.0**

(22) Date of filing: **11.09.84**

(51) Int. Cl.⁴: **C 07 C 5/27**
**C 07 C 15/02, B 01 J 29/06**

(30) Priority: **25.10.83 US 545330**

(43) Date of publication of application:
**15.05.85 Bulletin 85/20**

(84) Designated Contracting States:
**BE DE FR GB IT NL**

(71) Applicant: **MOBIL OIL CORPORATION**
**150 East 42nd Street**
**New York New York 10017(US)**

(72) Inventor: **Forbus, nancy Page**
**80 Colonial Drive**
**Newtown Pennsylvania 18940(US)**

(74) Representative: **West, Alan Harry**
**Mobil Court 3 Clements Inn**
**London WC2A 2EB(GB)**

(54) **Para-selective aromatic hydrocarbon conversions using silica bound zeolite catalysts.**

(57) A process is provided for enhancing the para-isomer content in the products resulting from the conversion of alkylbenzene compounds to dialkylbenzene compound mixtures. Examples of such conversions include the ethylation and disproportionation of toluene. The conversion takes place over a crystalline zeolite material combined with a matrix consisting essentially of amorphous silica as a binding material.

Croydon Printing Company Ltd.

PARA-SELECTIVE AROMATIC HYDROCARBON CONVERSIONS
USING SILICA BOUND ZEOLITE CATALYSTS

The present invention relates to the use of silica bound
zeolite catalyst compositions for the conversion of substituted
aromatic hydrocarbons to provide product mixtures enriched in the
para-(or 1,4-) dialkyl substituted benzene isomer.

Production of dialkyl substituted benzene compounds via
disproportionation, alkylation and/or transalkylation of aromatic
hydrocarbons is an important step in a number of commercial chemical
manufacturing processes. Such reactions can be carried out over a
variety of catalyst materials. Alkylation of aromatic hydrocarbons
utilizing crystalline aluminosilicate catalysts has, for example, been
described. U.S. Patent No. 2,904,607 to Mattox refers to alkylation
of aromatic hydrocarbons with an olefin in the presence of a
crystalline metallic aluminosilicate having uniform openings of about
6 to 15 Angstrom units. U.S. Patent No. 3,251,897 to Wise describes
alkylation of aromatic hydrocarbons in the presence of X or Y-type
crystalline aluminosilicate zeolites, specifically such type zeolites
wherein the cation is rare earth and/or hydrogen. U.S. Patent No.
3,751,504 to Keown et al. and U.S. Patent No. 3,751,506 to Burress
describe vapor phase alkylation of aromatic hydrocarbons with olefins,
e.g., benzene with ethylene, in the presence of a ZSM-5 type zeolite
catalyst.

The disproportionation of aromatic hydrocarbons in the
presence of zeolite catalysts has been described by Grandio et al in
the Oil and Gas Journal, Vol. 69, No. 48 (1971), U.S. Patent Nos.
3,126,422; 3,413,374, 3,598,878; 3,598,879 and 3,607,961 show
vapor-phase disproportionation of toluene over various catalysts.

In many of these prior art processes, the dialkylbenzene
product produced frequently contains more of the 1,3 isomer than of
the other two isomers. For example, xylene produced via the
conventional methylation of toluene has the equilibrium composition of approximately 24 percent of 1,4-, 54 percent of 1,3- and 22 percent of 1,2- isomer. Of the dialkylbenzene isomers, 1,3-dialkylbenzene is often the least desired product, with 1,2- and 1,4-dialkylbenzene being the more useful products. 1,4-Dimethylbenzene, for example, is of particular value, being useful in the manufacture of terephthalic acid which is an intermediate in the manufacture of synthetic fibers such as "Dacron"™. Furthermore, 1,4- methylethylbenzene, i.e., para-ethyltoluene (PET), is useful for subsequent conversion to para-methylstyrene, and for this purpose ethyltoluene products containing as much as 97% of the para isomer are required.

Mixtures of dialkylbenzene isomers, either alone or in further admixture with ethylbenzene, have previously been separated by expensive superfractionation and multistage refrigeration steps. Such processes, as will be realized, involve high operation costs and have a limited yield. Alternatively, various modified zeolite catalysts have been developed to alkylate or disproportionate toluene with a greater or lesser degree of selectivity to 1,4-dialkylbenzene isomers. Hence, U.S. Patents 3,972,832, 4,034,053, 4,128,592, and 4,137,195 disclose particular zeolite catalysts which have been treated with compounds of phosphorus and/or magnesium to increase para-selectivity of the catalysts. Para selective boron-containing zeolites are shown in U.S. Patent No. 4,067,920 and para-selective, antimony-containing zeolites in U.S. Patent No. 3,979,472. Similarly, U.S. Patent Nos. 3,965,208 and 4,117,026 disclose other modified zeolites useful for shape selective reactions.

In practicing any hydrocarbon conversion process, it is often useful to incorporate crystalline zeolites with a matrix comprising another material resistant to the temperature and other conditions employed in the process. Such matrix material is useful as a binder and imparts greater resistance to the catalyst for the severe temperature, pressure and reactant feed stream velocity conditions encountered in, for example, many cracking processes.

Notwithstanding the existence of bound zeolite catalysts having para-selective properties, there is a continuing need to develop additional types of catalytic materials which are highly para-selective when used for the conversion of aromatic compounds to dialkylbenzene products. Accordingly, it is an object of the present invention to provide bound zeolite catalyst compositions which promote the conversion of aromatics to produce mixtures containing an exceptionally high percentage, e.g., 97% by weight or more, for alkylation of toluene, of para-dialkylbenzene isomer.

It is a further object of the present invention to provide such highly para-selective catalysts without necessarily resorting to expensive and/or time consuming catalyst selectivation techniques such as steaming and/or precoking after each instance of catalyst regeneration.

It is a further object of the present invention to provide highly para-selective alkylation, transalkylation and disproportionation processes employing the modified zeolite catalysts described herein.

Thus the present invention is directed to a process for converting alkylbenzene compounds to a mixture of dialkylbenzenes enriched in the para-dialkylbenzene isomer. Such a process comprises contacting one or more of such alkylbenzene compounds under conversion conditions with a particular type of catalyst composite. The catalyst composite employed comprises a crystalline zeolite material having a silica to alumina ratio of at least 12 and a Constraint Index of 1 to 12. The catalyst composite also comprises a matrix material consisting essentially of amorphous silica.

As noted, the first essential component of the catalyst composite employed in the present process will comprise a zeolite of the ZSM-5 type. ZSM-5 type zeolites, i.e., zeolites of silica/alumina molar ratio greater than 12 and Constraint Index of 1 to 12 are well known. Their use as catalysts for the alkylation of aromatic compounds has, for example, been described in U.S. Patent 4,302,622. Crystalline zeolites of the type useful in the catalyst composites of the present invention include ZSM-5, ZSM-11, ZSM-12, ZSM-23, ZSM-35, ZSM-38, and ZSM-48, preferably ZSM-5, ZSM-12 and ZSM-23, with ZSM-5 being particularly preferred.

ZSM-5 is described in greater detail in U.S. Patent Nos. 3,702,886 and RE 29,948, which patents provide the X-ray diffraction pattern of the therein disclosed ZSM-5.

ZSM-11 is described in U.S. Patent No. 3,709,979, which discloses in particular the X-ray diffraction pattern of ZSM-11.

ZSM-12 is described in U.S. Patent No. 3,832,449, which discloses in particular the X-ray diffraction pattern of ZSM-12.

ZSM-23 is described in U.S. Patent No. 4,076,842, which discloses in particular the X-ray diffraction pattern of ZSM-23.

ZSM-35 is described in U.S. Patent No. 4,016,245, which discloses in particular the X-ray diffraction pattern of ZSM-35.

ZSM-38 is described in U.S. Patent No. 4,046,859, which discloses in particular the X-ray diffraction pattern of ZSM-38.

ZSM-48 is described in U.S. Patent No. 4,375,573 and European Patent Publication EP-A-0015132, which disclose in particular the X-ray diffraction pattern of ZSM-48.

The specific zeolites described, when prepared in the presence of organic cations, are catalytically inactive, possibly because the intracrystalline free space is occupied by organic cations from the forming solutions. They may be activated by heating in an inert atmosphere at 1000°F (538°C) for 1 hour, for example, followed by base exchange with ammonium salts followed by calcination at 1000°F (538°C) in air. The presence of organic cations in the forming solution may not be absolutely essential to the formation of this type zeolite; however, the presence of these cations does appear to favor the formation of this special type of zeolite. More generally, it is desirable to activate this type catalyst by base exchange with ammonium salts followed by calcination in air at about 1000°F (538°C) for from about 15 minutes to about 24 hours.

Thus when synthesized in the alkali metal form, the zeolite is conveniently converted to the hydrogen form, generally by intermediate formation of the ammonium form as a result of ammonium ion exchange and calcination of the ammonium form as a result of ammonium ion exchange and calcination of the ammonium form to yield the hydrogen form. In addition to the hydrogen form, other forms of the zeolite wherein the original alkali metal has been reduced to less
than about 1.5 percent by weight may be used.  In this manner, the
original alkali metal of the zeolite may be replaced by ion exchange
with other suitable ions of Groups IB to VIII of the Periodic Table,
including by way of example, nickel, copper, zinc, palladium, calcium
or rare earth metals.

Advantageously the zeolite component of the catalyst
composites used in the present process can comprise from about 1
percent to 99 percent by weight of the composite on an anhydrous
basis.  More preferably the zeolite will comprise from about 5 percent
to 80 percent by weight of the catalyst composite on an anhydrous
basis.

The second essential component of the catalyst composite used
in the alkylbenzene conversion process of the present invention is a
matrix material consisting essentially of amorphous silica.  Matrix
materials have, of course, conventionally been employed in catalyst
composites to function as a binder for the active catalytic component,
e.g., a zeolite material.  Matrix materials thus serve to impart
greater physical strength and stability to catalysts which may
encounter severe temperatures, pressures, reactant feedstream
velocities and catalyst circulation rates in use.  It has been
discovered, however, that the particular matrix material amorphous
silica, when combined with the zeolites hereinbefore described,
provide catalysts which are especially effective for the
para-selective conversion of alkylbenzene compounds in accordance with
the process of the present invention.

The relative proportions of zeolite component and silica gel
matrix component may vary widely.  Preferably, on an anhydrous basis,
the amorphous silica will comprise from about 1 percent to about 99
percent by weight of the catalyst composition, more preferably from
about 20 percent to 95 percent by weight of the catalyst composition.
Typically the matrix component of the catalyst, on an anhydrous basis,
will be substantially all amorphous silica.  However, the silica may
be combined with as much as 50 percent by weight of matrix of
additional substantially catalytically inactive binder materials, for example, combined with the silica in a cogel.  For purposes of the present invention, the matrix should be substantially free of catalytically active binder materials such as alumina.

The catalyst compositions used in the present invention can be prepared in any manner that provides an intimate admixture of the zeolite component and silica-based matrix material.  Typically the zeolite and matrix are thoroughly physically admixed, for example, by coextruding the zeolite and the matrix material.

When the catalyst compositions of the type hereinbefore described are to be used for the conversion of aromatic compounds, the para-selective properties of such catalysts can preferably be enhanced in known manner by the treatment of such catalysts with oxides of a number of elements.  Most commonly such catalysts can be treated with phosphorus and/or magnesium compounds in the manner described in U.S. Patent Nos.  3,894,104; 4,049,573; 4,086,287; and 4,128,592.

Phosphorus, for example, can be incorporated into such zeolites in the form of phosphorus oxide in an amount of from about 0.25% to about 25% by weight of the catalyst composition.  Such incorporation can be readily effected by contacting the zeolite composite with a solution of an appropriate phosphorus compound, followed by drying and calcining to convert the phosphorus compound to its oxide form.  The amount of magnesium oxide incorporated into the zeolite may also be from about 0.25% to about 25% by weight of the catalyst composition.

In addition to treatment of the zeolite composites with phosphorus and/or magnesium as hereinbefore described in detail, such zeolites may also be treated with a variety of other oxide materials to enhance para-selectivity.  Such oxide materials include oxides of boron (U.S. 4,067,920); antimony (U.S. 3,979,472); beryllium (U.S. 4,260,843); Group VIIA metals (U.S. 4,275,256); alkaline earth metals (U.S. 4,288,647); Group IB metals (U.S. 4,276,438); Group IVB metals (U.S. 4,278,827); Group VIA metals (U.S. 4,259,537); Group IA elements (U.S. 4,329,533); cadmium (European Patent Application EP-A-38116); iron and/or cobalt (European Patent Application EP-A-40463); Group IIIB metals (U.S. 4,276,437); Group IVA metals (U.S. 4,302,620); Group VA metals (U.S. 4,302,621); and Group IIIA elements (U.S. 4,302,622).

Additional catalyst modifying procedures which may also optionally be employed to enhance catalyst para-selectivity include precoking and steaming, or combinations thereof.

Steaming entails contact of the zeolite with an atmosphere containing from about 5 to about 100 percent steam at a temperature of from about 250°C to about 1000°C for a period of between about 15 minutes and about 100 hours and under pressures ranging from sub-atmospheric to several hundred atmospheres. Preferably, steam treatment is effected at a temperature of between about 400°C and about 700°C for a period of between about 1 and about 24 hours.

Precoking of the catalyst serves to deposit a coating of between about 2 and about 75, and preferably between about 15 and about 75, weight percent of coke thereon to enhance catalyst selectivity. Precoking can be accomplished by contacting the catalyst with a hydrocarbon charge, e.g., toluene, under high severity conditions or alternatively at a reduced hydrogen to hydrocarbon relative concentration, i.e., 0 to 1 mole ratio of hydrogen to hydrocarbon, for a sufficient time to deposit the desired amount of coke thereon.

The silica bound zeolite catalysts of the present invention are advantageously used to promote conversion of alkylbenzene compounds to provide dialkyl-substituted benzene product mixtures which are highly enriched, in the para-dialkyl substituted benzene isomer. Aromatic compound conversion of this type includes alkylation, transalkylation and disproportionation.

Alkylation of alkylbenzene compounds in the presence of the above-described catalyst is effected by contact of the alkylbenzene with an alkylating agent. A particularly preferred embodiment involves the alkylation of toluene wherein he alkylating agents employed comprise methanol or other well known methylating agents or ethylene. Alkylation reactions can be carried out at a temperature of between about 250°C and about 750°C, preferably between about 300°C and 650°C. At higher temperatures, the zeolites of high silica/alumina ratio are preferred. For example, ZSM-5 having a $SiO_2/Al_2O_3$ ratio of 30 and upwards is exceptionally stable at high temperatures. The reaction generally takes place at atmospheric pressure, but pressures within the approximate range of $10^5$ N/m$^2$ to $10^7$ N/m$^2$ (1-100 atmospheres) may be employed.

Some non-limiting examples of suitable alkylating agents would include olefins such as, for example, ethylene, propylene, butene, decene and dodecene, as well as formaldehyde, alkyl halides and alcohols, the alkyl portion thereof having from 1 to 16 carbon atoms. Numerous other aliphatic compounds having at least one reactive alkyl radical may be utilized as alkylating agents.

Alkylbenzene compounds which may be selectively alkylated as described herein would include any alkylatable alkylbenzene such as, for example, ethylbenzene, toluene, propylbenzene, isopropylbenzene, methylphenol, methylchlorobenzene, or substantially any mono- or di-substituted alkylbenzenes which are alkylatable in the 4-position of the aromatic ring.

The molar ratio of alkylating agent to aromatic compound may generally between about 0.05 and about 5. For instance, when methanol is employed as the methylating agent and toluene is the aromatic, a suitable molar ratio of methanol to toluene has been found to be approximately 0.1 to 1.0 mole of methanol per mole of toluene. When ethylene is employed as the alkylating agent and toluene is the aromatic, a suitable molar ratio of ethylene to toluene is approximately 0.05 to 2.5 moles of ethylene per mole of toluene.

Alkylation can suitably be accomplished utilizing a feed weight hourly space velocity (WHSV) of between about 1 and about 1000, and preferably between about 1 and about 200. The reaction product, consisting predominantly of the 1,4-dialkyl isomer, e.g. 1,4-dimethylbenzene, or a mixture of the 1,4- and 1,3- isomers together with comparatively smaller amounts of 1,2-dialkylbenzene isomer, may be separated by any suitable means. Such means may include, for example, passing the reaction product stream through a water condenser and subsequently passing the organic phase through a column in which chromatographic separation of the aromatic isomers is accomplished. Alkylation using the silica-bound zeolite catalysts of the present invention can provide product mixtures containing at least 90% or even 95% or more by weight of the para-dialkylbenzene isomer.

When transalkylation is to be accomplished, transalkylating agents are alkyl or polyalkyl aromatic hydrocarbons wherein alkyl may be composed of from 1 to about 5 carbon atoms, such as, for example, toluene, xylene, trimethylbenzene, triethylbenzene, dimethylethylbenzene, ethylbenzene, diethylbenzene, ethyltoluene, and so forth.

Another process embodiment of this invention relates to the selective disproportionation of alkylated aromatic compounds to produce dialkylbenzenes wherein the yield of 1,4-dialkyl isomer is in excess of the normal equilibrium concentration. In this context, it should be noted that disproportionation is a special case of transalkylation in which the alkylatable hydrocarbon and the transalkylating agent are the same compound, for example when toluene serves as the donor and acceptor of a transferred methyl group to produce benzene and xylene.

The transalkylation and disproportionation reactions can be carried out by contacting the reactants with the silica-bound zeolite catalyst at a temperature of between about 250°C and 750°C at a pressure of between atmospheric ($10^5$ N/m$^2$) and about 100 atmospheres ($10^7$ N/m$^2$). The reactant feed WHSV will normally fall within the range of about 0.1 to about 50. Preferred alkylated aromatic compounds suitable for utilization in the disproportionation embodiment comprise toluene, ethylbenzene, propylbenzene or substantially any mono-substituted alkylbenzene. These aromatic compounds are selectively converted to, respectively, 1,4-dimethylbenzene, 1,4-diethylbenzene, 1,4-dipropylbenzene, or other 1,4-dialkylbenzene, as appropriate, with benzene being a primary side product in each instance. The product can be recovered from the reactor effluent by conventional means, such as distillation, to remove the desired products of benzene and dialkylbenzene, and any unreacted aromatic component is recycled for further reaction.

The alkylbenzene conversion processes described herein may be carried out as a batch type, semi-continuous or continuous operation utilizing a fixed or moving bed catalyst system. The catalyst after use in a moving bed reactor is conducted to a regeneration zone wherein coke is burned from the catalyst in an oxygen-containing atmosphere, e.g. air, at an elevated temperature, after which the regenerated catalyst is recycled to the conversion zone for further contact with the charge stock. In a fixed bed reactor, regeneration is carried out in a conventional manner where an inert gas containing a small amount of oxygen (0.5-2%) is used to burn the coke in a controlled manner so as to limit the temperature to a maximum of around 500°-550°C.

The following examples will serve to illustrate certain specific embodiments of the hereindisclosed invention. These examples should not, however, be construed as limiting the scope of the invention, as there are many variations which may be made thereon without departing from the spirit of the disclosed invention, as those of skill in the art will recognize.

## EXAMPLE 1

A sample of a ZSM-5 catalyst having a shortest diffusional path of 2 microns, designated herein as zeolite A, was extruded with both silica and alumina as binder. These two catalysts were designated zeolite A/silica and zeolite A/alumina, respectively. About 2.2 gms. of each of these catalysts were centered in a quartz microreactor. Low surface area quartz chips were used to center the catalyst and fill void spaces. Toluene is passed over the catalyst at a WHSV=8.6 $hr^{-1}$. Ethylene is metered to the reactor at a WHSV=0.5 $hr^{-1}$. The temperature is maintained at 400°C. After a 15 minute run, a sample is collected over a period of 5 minutes. The temperature is then increased to 450°C and the procedure is repeated. The performance of these two catalysts is shown in Table 1. As can be seen from Table 1, extruding zeolite A in a silica binder gives a catalyst that exhibits a higher para-selectivity than when the same crystals are extruded with alumina as the binder.

Table 1

Effect of Binder on Performance of Zeolite A
for Selective Alkylation of Toluene with Ethylene

| Binder | Temp., °C | Toluene Conv. | % PET/ET |
|--------|-----------|---------------|----------|
| alumina | 400° | 23.9 | 33.2 |
| silica | 400° | 18.6 | 42.8 |
| alumina | 450° | 20.4 | 29.9 |
| silica | 450° | 17.2 | 39.0 |

EXAMPLE 2

In replacement of zeolite A, samples of a ZSM-5 catalyst having a shortest diffusional path of 5 microns, designated herein as zeolite B, were extruded in both silica and alumina binder and were designated zeolite B/silica and zeolite B/alumina, respectively. These catalysts were tested for the selective alkylation of toluene with ethylene to produce p-ethyltoluene under the same conditions described in Example 1. Performance of this catalyst is shown in Table 2. Again, it can be seen that use of a silica binder results in a significantly higher para-selectivity than is observed for the same zeolite B in alumina binder.

Table 2

Effect of Binder on Performance of Zeolite B
for Selective Alkylation of Toluene with Ethylene

| Binder | Temp., °C | Toluene Conv. | % PET/ET |
|--------|-----------|---------------|----------|
| alumina | 400° | 17.0 | 70.0 |
| silica | 400° | 15 9 | 89.6 |
| alumina | 450° | 14.5 | 59.4 |
| silica | 450° | 12.3 | 85 6 |

**0141514**

## EXAMPLE 3

The catalysts described in Example 1, i.e. zeolite A/alumina and zeolite A/silica, were tested for performance in the para-selective alkylation of toluene with ethylene to produce p-ethyltoluene at superatmospheric pressure in the presence of hydrogen. For reactions carried out above atmospheric pressure, the vapor phase reactor was constructed entirely of 316 or 321 stainless steel. The reactor dimensions were 1-1/4 inch (31.75 mm) od, 5/8 inch (15.9 mm) id x 21-5/8 inches (55 cm) long and rated to operate at 5000 psig (3.5 x $10^4$ kPa) at 650°F (343°C). It was fitted with a metal, spiral insert in the top section which functioned as the preheater. The catalyst section contained a 3/16 x 1/8 inch (4.8 x 3.2 mm) centered thermowell. A three-zone furnace with independent controls was used to obtain the desired temperature. Three grams of the catalyst were centered in the middle zone with low surface area quartz chips to prevent void spaces. Liquid toluene was pumped at the rate of 167 ml/hr to the top of the reactor where it mixed with ethylene and hydrogen at rates measured by calibrated mass flow meters. The ethylene and hydrogen feed rates were 83 cc/min and 375 cc/min, respectively. The catalysts were screened at 435°C and 250 psig (1825 kPa). After 1.0 hr on stream, gas and liquid samples were collected. Catalyst performance at these conditions is given in Table 3. Table 3 shows that increased para-selectivity with silica binder is also observed when the reaction is carried out above atmospheric pressure in the presence of hydrogen.

Table 3

Effect of Binder on Performance of Zeolite A Under

$H_2$ Pressure for Alkylation of Toluene with Ethylene

| Binder | Toluene Conv. | % PET/ET |
|--------|--------------|----------|
| alumina | 18.5 | 35.1 |
| silica | 19.0 | 44.1 |

## EXAMPLE 4

The catalysts described in Example 2 were also screened for performance in the selective alkylation of toluene with ethylene to produce p-ethyltoluene above atmospheric pressure in the presence of hydrogen. The same conditions as described in Example 3 were used. Table 4 summarizes these results. Consistent with atmospheric pressure results on these zeolite B crystals, the use of a silica binder gives a more para-selective catalyst for the selective alkylation of toluene with ethylene at superatmospheric pressure in the presence of hydrogen.

### Table 4

### Effect of Binder on Performance of Zeolite B
### for Alkylation of Toluene with Ethylene

| Binder | Toluene Conv. | % PET/ET |
|--------|---------------|----------|
| alumina | 15.0 | 71.4 |
| silica | 11.2 | 90.4 |

## EXAMPLE 5

A sample of zeolite A was extruded with both silica and alumina as binder. These two catalysts were designated zeolite A/silica and zeolite A/alumina, respectively. 2.2 gms of each of these catalysts were centered in a quartz microreactor. Low surface area quartz chips were used to center the catalyst and fill void spaces. The temperature was adjusted to 450°C and toluene was passed over the catalyst at WHSV=3.6 $hr^{-1}$. After 15 minutes, a liquid sample was collected for a total of 5 minutes. The temperature was then increased to 500°, 550° and 600° repeating the same sample collecting procedure. After the sample was collected at 600°C, the catalyst was cooled to 500°C under $N_2$, then calcined in air for 2 hrs. before use in the next set of reactions. Following the calcination, the temperature was lowered to 400°C and a mixture of toluene and methanol was passed over the catalyst at a toluene WHSV=10.3 $hr^{-1}$ and methanol WHSV=0.9 $hr^{-1}$. The same sampling

procedure as described for the toluene disproportionation reaction was followed. Catalyst performance was evaluated at 400°, 500° and 600°C. The results of these screenings are summarized in Tables 5 and 6.


Table 5

Effect of Binder for Para-Selectivity in Selective
Toluene Disproportionation with Zeolite A

| Binder | Temp., °C | Toluene Conv. | % PX/xylenes |
|--------|-----------|---------------|--------------|
| alumina | 450° | 14.6 | 27.3 |
| silica | 450° | 8 8 | 29.6 |
| | | | |
| alumina | 500° | 30.0 | 26.2 |
| silica | 500° | 22.8 | 28.0 |
| | | | |
| alumina | 550° | 46.5 | 24.4 |
| silica | 550° | 40.0 | 26.2 |
| | | | |
| alumina | 600° | 53.3 | 22.6 |
| silica | 600° | 49.4 | 24.9 |

Table 6

Effect of Binder on Para-Selectivity in

Toluene + Methanol Reaction with Zeolite A

| Binder | Temp., °C | Toluene Conv. | % PX/xylenes |
|---|---|---|---|
| alumina | 400° | 14.5 | 33.1 |
| silica | 400° | 13.2 | 45.6 |
| alumina | 500° | 19.6 | 28.1 |
| silica | 500° | 21.1 | 35.7 |
| alumina | 600° | 35.0 | 29.0 |
| silica | 600° | 35.2 | 33.0 |

EXAMPLE 6

Samples of zeolite B were extruded with both silica and alumina binder. These extrudates were designated zeolite B/silica and zeolite B/alumina, respectively. These catalysts were tested for performance in the production of p-xylene both by selective toluene disproportionation and by alkylation of toluene with methanol using the procedure described in Example 5. Results of these runs are shown in Tables 7 and 8.

F-2458 -16- **0141514**

## Table 7

### Effect of Binder on Para-Selectivity in the Selective Toluene Disproportionation with Zeolite B

| Binder | Temp., °C | Toluene Conv. | % PX/xylenes |
|---|---|---|---|
| alumina | 450° | 5.9 | 45.6 |
| silica | 450° | 8.4 | 55.1 |
| alumina | 500° | 16.4 | 41.6 |
| silica | 500° | 21.6 | 52.9 |
| alumina | 550° | 31.8 | 37.1 |
| silica | 550° | 33.9 | 52.4 |
| alumina | 600° | 40.7 | 35.6 |
| silica | 600° | 26.4 | 56.8 |

## Table 8

### Effect of Binder on Para-Selectivity in Alkylation of Toluene with Methanol Using Zeolite B

| Binder | Temp., °C | Toluene Conv. | % PX/xylenes |
|---|---|---|---|
| alumina | 400° | 9.8 | 73.2 |
| silica | 400° | 9.9 | 90.8 |
| alumina | 500° | 7.1 | 59.9 |
| silica | 500° | 13.6 | 85.5 |
| alumina | 600° | 12.6 | 54.9 |
| silica | 600° | 15.1 | 82.3 |

## EXAMPLE 7

It is known that impregnation of para-selective catalysts with metal salts that form difficultly reducible oxides results in catalysts with even higher para-selectivity. The catalyst designated zeolite B/silica was impregnated with a solution containing 20% $Mg(NO_3)_2$. Following calcination, it was found to contain 2.2% Mg. The catalyst designated zeolite B/alumina was impregnated with a solution of 50% $Mg(NO_3)_2$ using the same procedure. After calcination, this catalyst was found to contain 2.9% Mg. Both catalysts were then tested for STDP and toluene + methanol reactions using the procedure described in Example 5. These results are shown in Tables 9 and 10.

Table 9

Effect of Binder on Para-Selectivity

in the STDP Reaction Using Mg-Zeolite B

| Binder | Temp., °C | Toluene Conv. | % PX/xylenes |
|--------|-----------|---------------|--------------|
| alumina | 450° | 5.0 | 92.4 |
| silica | 450° | 4.4 | 95 7 |
| | | | |
| alumina | 500° | 12.7 | 90.6 |
| silica | 500° | 10.8 | 94.4 |
| | | | |
| alumina | 550° | 21.7 | 87.5 |
| silica | 550° | 20.2 | 91 8 |
| | | | |
| alumina | 600° | 25.2 | 83.6 |
| silica | 600° | 24.2 | 89 9 |

## Table 10

### Effect of Binder on Para-Selectivity in Alkylation
### of Toluene with Methanol Using Mg-Zeolite B

| Binder | Temp., °C | Toluene Conv. | % PX/xylenes |
|--------|-----------|---------------|--------------|
| alumina | 400° | 8 7 | 97.4 |
| silica | 400° | 10.3 | 100 |
| alumina | 500° | 12.5 | 95.0 |
| silica | 500° | 10.5 | 97.4 |
| alumina | 600° | 13.4 | 91.6 |
| silica | 600° | 13.5 | 95.2 |

As can be seen from Tables 9 and 10, both the STDP and toluene + methanol reactions, the catalyst extruded with a silica binder exhibits a higher para-selectivity than does the same catalyst in an alumina binder. This is in spite of the fact that the alumina bound catalyst actually has a slightly higher level of magnesium, and so, should exhibit a somewhat higher para selectivity, all other conditions being equal.

### EXAMPLE 8

The rate of o-xylene sorption is a standard measurement commonly used to determine the relative diffusion rates in catalysts used for para-selective reactions. This diffusion rate (reported as $D/r^2$) can be used to predict which catalysts will exhibit a high para-selectivity. Catalysts which show a higher rate of o-xylene diffusion will have a lower observed para-selectivity for alkylation or selective disproportionation reactions. The relative rates of diffusion for these four catalysts as well as for the pure zeolite materials have been measured and are given in Table 11.

Table 11

| Catalyst | $D/r^2$ $(sec^{-1})$ |
|---|---|
| Zeolite B (pure) | $2.2 \times 10^{-6}$ |
| Zeolite B/silica | $0.7 \times 10^{-6}$ |
| Zeolite B/alumina | $0.8 \times 10^{-6}$ |
| Zeolite A (pure) | $4.5 \times 10^{-6}$ |
| Zeolite A/silica | $1.9 \times 10^{-6}$ |
| Zeolite A/alumina | $3.3 \times 10^{-6}$ |

The differences between the two crystals, zeolite A and zeolite B, can be explained based on differences in the crystal sizes. Zeolite B has a shortest diffusional path of 5 microns compared to only 2 microns for zeolite A. This explains differences between observed para-selectivities for the two catalysts and is a totally expected result. However, what is not obvious is the unexpected result that for the same size ZSM-5 crystal, extrusion in silica results in a catalyst with a lower rate of diffusion that is obtained for the same ZSM-5 sample in an alumina binder. However, these differences in rate of diffusion with binder can explain, in part, observed differences in para-selectivity for the two binders.

EXAMPLE 9

Para-ethyltoluene (PET) was isomerized over 2.2 gms extrudates of zeolite A/silica, zeolite A/alumina, zeolite B/silica, zeolite B/alumina, pure silica (i.e. having no zeolite) and pure alumina (i.e. having no zeolite). Ethyltoluenes consisting of 97% para-isomer and 3% meta-isomer were passed over the extrudates at the rate of 9.15 ml/hr with a $N_2$ co-feed of 25 cc/min at a temperature of 280°C. This temperature was chosen so that the more active catalysts gave appreciable amounts of isomerization but cracking (which would complicate interpretation of the isomerization results) was negligible. Compositions of the resulting liquid products are given in Table 12.

Table 12

| Catalyst | Zeolite B | | Zeolite A | | Pure | | Feed |
|---|---|---|---|---|---|---|---|
| | silica | alumina | silica | alumina | silica | alumina | |
| PET | 96.3 | 93.3 | 89.4 | 76.4 | 97.1 | 97 1 | 97.0 |
| MET | 3.7 | 6.7 | 10.6 | 23.4 | 2.9 | 2.9 | 3.0 |
| OET | -- | -- | -- | 0.2 | -- | -- | -- |
| % ET in liquid | 98 9 | 99.2 | 98.9 | 98.0 | 99.9 | 99.9 | 99.9 |

As these results demonstrate, there is a difference in the amount of PET isomerization observed for the same ZSM-5 crystal in the two binders. There is more apparent isomerization for a zeolite extruded in alumina. However, as can be seen from results obtained with the pure binder materials, neither has any activity for isomerization under these conditions. In addition, the fact that very little or no ortho isomer is produced suggests that this isomerization is occurring inside the zeolite itself as opposed to at a non-constrained site. Again, the relative rates of diffusion appear to be determining the observed isomer distributions. That the rate of diffusion should change as the binder material is changed is an unexpected result.

CLAIMS

1. A process for converting alkylbenzene compounds to a mixture of dialkylbenzenes enriched in the para-dialkylbenzene isomer, which process comprises contacting one or more of said alkylbenzene compounds under conversion conditions with a catalyst composite comprising a crystalline zeolite material having a silica to alumina ratio of at least 12 and Constraint Index of 1 to 12 and a matrix material consisting essentially of amorphous silica.

2. A process according to Claim 1 wherein the crystalline zeolite material comprises from about 5 percent to 80 percent by weight of a composite and the amorphous silica comprises from about 20 percent to 95 percent by weight of the composite.

3. A process according to Claim 1 or Claim 2 wherein the crystalline zeolite material used in the catalyst composite is selected from ZSM-5, ZSM-11, ZSM-12, ZSM-23, ZSM-35, ZSM-38 or ZSM-48.

4. A process according to any of Claims 1 to 3 wherein the catalyst composite also comprises from about 0.25 percent to 25 percent by weight of a modifying oxide selected from magnesium oxide, phosphorous oxides or mixtures of said oxides.

5. A process according to any of Claims 1 to 4 wherein the conversion of alkylbenzene compounds comprises alkylation of a monoalkylbenzene compound with an alkylating agent.

6. A process according to Claim 5 wherein said alkylating agent contains from 1 to 16 carbon atoms and is selected from olefins, alkyl halides or alcohols.

7. A process according to Claim 5 wherein said monoalkylbenzene compound is toluene and said alkylating agent is ethylene.

8. A process according to any of Claims 1 to 4 wherein the conversion of alkylbenzene compounds comprises transalkylation.

9. A process according to any of Claims 1 to 4 wherein the conversion of alkylbenzene compounds comprises disproportionation of a monoalkylbenzene compound to produce benzene and a mixture of dialkylbenzene isomers.

10.  A process according to Claim 9 wherein said monoalkylbenzene compound is toluene and said dialkylbenzene isomers are xylene isomers.

4389H/0147D